# EUROPEAN PATENT APPLICATION

(11) **EP 1 514 938 A1**
(43) Date of publication of application: **16.03.2005**
(21) Application number: 04004011.5
(22) Date of filing: 23.02.2004
(51) Int. Cl.: C12N 15/52, C12N 9/00

(54) **Heat-resistant DNA ligase**

(30) Priority: 24.02.2003 JP 2003045224
(71) Applicant: National Institute of Advanced Industrial Science and Technology, Tokyo 100-8921 (JP)
(72) Inventor: Ishikawa, Kazuhiko, Osaka 563-8577 (JP); Joen, Sung-Jong c/o Nat. Inst. of Advanced Ind., Ikeda, Osaka 563-8577 (JP)
(74) Representative: Mütschele, Thomas, Dr. Dipl.-Chem.

(57) **Abstract**

A heat-resistant ligase isolated from *Aeropyrum pernix*, and homologues thereof, the activity of which is not substantially decreased by heat treatment at 100°C, is disclosed.

## Description

### Field of the Invention

The present invention relates to a heat-resistant ligase used in genetic engineering, such as in recombination and amplification of DNA.

### Background of the Invention

DNA ligase is an enzyme having the ability to link DNA chains by forming a phosphodiester linkage between a 3'-OH group and 5'-phosphate group of DNA. This enzyme is essential for gene recombination technologies. A heat liable DNA ligase derived from bacteria and phage is usually used for gene recombination.

DNA ligase is also used in a specific nucleotide amplification reaction known as a ligase-mediated coupling - reaction (LCR: Landergen U. (1988), Science. 241, 1077-1080; Barany, -F. (1991), PCR Methods and Applications 1:5-16; Marsh E. (1992), Strategiens 5: 73-76). LCR is a method by which known nucleotide sequences are amplified or detected by a temperature-cycling reaction using a heat-resistant DNA ligase. In more detail, LCR is performed by mixing two adjoining oligonucleotides complimentary to a desired nucleotide sequence, an oligonucleotide complimentary to each nucleotide and heat-resistant DNA ligase, and by repeated temperature cycling comprising denaturation at a high temperature, annealing at a low temperature, and a coupling reaction. A Pfu DNA ligase derived from *Pyrococcus friosus* has been used for LCR.

Another heat-resistant DNA ligase is isolated from hyperthermophilic archaea KOD-1 strains, and has an optimum temperature of 80°C and displays activity even at 100°C (Japanese Unexamined Patent Publication Application No. 12-308494 (paragraph Nos. 0007 and 0056)).

However, because the DNA ligase used in LCR is subjected to repeated heating and cooling of the sample, it desirably maintains its original activity at a high temperature, even after heat treatment at a high temperature for a long period of time.

The main object of the present invention is to provide a DNA ligase that maintains high activity even after heat treatment at a high temperature.

### Summary of the Invention

The inventors have found, through intensive studies to achieve the above object, that a DNA ligase obtained by cloning DNA ligase genes from the primitive extreme thermophile *Aeropyrum pernix* K1 strain has excellent heat resistance since the activity thereof is not substantially decreased by heat-treating at 100°C for 1 hour.

The completed invention based on the discovery above provides the following heat-resistant DNA ligase:
1. A heat-resistant DNA ligase having activity that is not substantially decreased by heat treatment at 100°C for 1 hour.
2. A heat-resistant DNA ligase described in 1. above, having the following properties;
   (a) an optimum temperature of 70°C or more;
   (b) utilization of ATP or ADP as a cofactor; and
   (c) utilization of Mg²⁺, Mn²⁺, Ca²⁺ or Co²⁺ as a cofactor.
3. A heat-resistant DNA ligase described in 1. and 2. above that is an enzyme derived from *Aeropyrum pernix.*
4. A polypeptide comprising:
   (a) a polypeptide having an amino acid sequence represented by SEQ ID NO:2.[accession no APE1094 of *A. pernix];* or
   (b) a polypeptide having an amino acid sequence comprising one or a plurality of deleted, substituted or added amino acids in SEQ ID NO:2, the DNA ligase activity thereof being not substantially decreased by heat treatment at 100°C for 1 hour.

   4.1. A polypeptide described in 4., wherein the polypeptide has at least about 90% sequence homology with the amino acid sequence set forth in SEQ ID NO:2.
5. A polynucleotide comprising;
   (a) a polynucleotide having a base sequence represented by SEQ ID NO:1 (accession no APE1094 of *A. pernix);*
   (b) a polynucleotide that hybridizes with the complement of a polynucleotide having a nucleotide sequence represented by SEQ ID NO:1 under stringent conditions, the polynucleotide encoding a heat-resistant DNA ligase having an activity being not substantially decreased by heat treatment at 100°C for 1 hour;
   (c) a polynucleotide that encodes a polypeptide having at least about 90% sequence homology with the polypeptide set forth in SEQ ID NO:2, wherein the polypeptide encoded by the polynucleotide is a heat-resistant DNA ligase having activity that is not substantially decreased by heat treatment at 100°C for 1 hour; or
   (d) a polynucleotide encoding the polypeptide according to 4. above.
6. A recombinant vector containing any of the polynucleotides described above.
7. -A host cell transformed with the vector described above.
8. A method for producing and retrieving a heat-resistant DNA ligase from the cultivated transformant described above.

### Brief Description of the Drawings

Fig. 1 is a graph showing the optimum temperature of the DNA ligase derived from the *Aeropyrum pernix* K-1 strain.
Fig. 2 is a graph showing the residual activity ratio after heat treatment of the DNA ligase derived from the *Aeropyrum pernix* K-1 strain.

### Detailed Description of the Invention

The present invention will be described in detail hereinafter.

### I. DNA ligase

### Heat resistance

The DNA ligase of the present invention is an enzyme having excellent heat resistance, and the DNA ligase activity is not substantially decreased by heat treatment at 100°C for 1 hour. The phrase "the activity is not substantially decreased" as used in the present invention includes a residual activity ratio of about 90% or more of the activity before the heat treatment.

The DNA ligase of the present invention is preferably an enzyme whose activity is not substantially decreased after heat treatment at 100°C for 2 hours, and more preferably an enzyme whose activity is not substantially decreased after heat treatment at 100°C for 12 hours. It is further preferable that the enzyme has a residual activity ratio of about 80% or more by heat treatment at 110°C for 10 minutes.

The DNA ligase of the present invention is preferably an enzyme having an optimum temperature of 70°C or more obtained by measuring the initial reaction rate of the enzyme, although the temperature differs depending on the kind of buffer solutions used for the enzyme reaction.

The DNA ligase activity is measured by a nick translation assay or cohesive end ligation assay according to the items in the examples of the present invention.

### Stability at room temperature

The DNA ligase of the present invention naturally has excellent stability at room temperature. Preferably, the activity of the DNA ligase of the present invention is not substantially decreased by allowing the enzyme to stand at room temperature (25°C) for 10 days or more.

### Organic solvent resistance

The DNA ligase of the present invention is resistant to organic solvents. For example, the enzyme preferably exhibits activity in a buffer solution containing 20% by volume or more of an organic solvent such as ethanol, butanol, tetrahydrofuran and ethyl acetate. The upper limit of the volume ratio of the organic solvent in the buffer solution for the DNA ligase of the present invention capable of exhibiting enzymatic activity is in a concentration range that does not cause precipitation of the enzyme protein.

### Cofactor

The heat-resistant DNA ligase of the present invention usually requires ATP or NAD as a cofactor. Both ATP and ADP are preferably used as the cofactors in the DNA ligase of the present invention.

While the DNA ligase of the present invention requires divalent cations, any one of Mg²⁺, Mn²⁺, Ca²⁺ and Co²⁺ is preferable as the divalent cations.

### Production bacteria

While the origin of DNA ligase of the present invention is not particularly restricted, examples of the enzyme include those produced by *Aeropyrum, Pyrococcus, Thermococcus, Syfolobus; Thermoplasma, Thermoproteus, Mastigocladus, Bacillus, Synechococcus* and *Thermus* species. The enzymes produced by the super thermophile archaebacteriun *Aeropyrum pernixis* are particularly preferable.

### Amino acid sequence

The heat-resistant DNA ligase of the present invention comprises polypeptides having the following amino acid sequences:
(1) a polypeptide having an amino acid sequence represented by SEQ ID NO: 2; and
(2) a polypeptide having an amino acid sequence comprising one or a plurality of deleted, substituted or added amino acids in SEQ ID NO:2 having a DNA ligase activity that is not substantially decreased by a heat treatment at 100°C for 1 hour.

While the polypeptide (2) is preferably a polypeptide having an amino acid sequence comprising (a) 1 to about 200, particularly (b) 1 to about 100, deleted, substituted or added amino acids in the amino acid sequence represented by SEQ ID NO:2, the amino acid sequence in SEQ ID NO:2 may be modified in the range of 30% or less of the total amino acids so long as the amino acids belong to a non-conservative region between the DNA ligase regions.

Thus, the polypeptide (2) includes polypeptides having at least about 70%, 75%, 80%, 85%, 90%, 91%, 92%, 93%, 94%, 95%, 96%, 97%, 98%, or 99% sequence homology with the polypeptide of SEQ ID NO:2. Preferably, the amino acid changes in the homologous polypeptides occur in regions of the polypeptide of SEQ ID NO:2 that are not conserved among known DNA ligase polypeptides.

Actually, the amino acids may be substituted with other amino acids having properties resembling the amino acids before substitution in terms of polarity, charge, solubility and hydrophilicity/hydrophobicity in view of retention of the protein structure. For example, glycine, alanine, valine, leucine, isoleucine and proline are classified as non-polar amino acids; serine, threonine, cysteine, methionine, asparagine and glutamine are classified as polar amino acids; lysine, arginine and histidine are classified as basic amino acids; and aspartic acid and glutamic acid are classified as acidic amino acids. Accordingly, these amino acids may be selectively substituted with the amino acids belonging to the same group.

Since the three-dimensional structure of the polypeptide is hardly changed when the size of the side chain is reduced by substitution of an amino acid, and the activity of the enzyme is hardly changed, the polypeptide described in (2) above can be obtained by such substitution.

The DNA ligase of the present invention can be obtained, for example, by cultivating the bacteria that produce the enzyme, and by retrieving or purifying the enzyme from a solution of pulverized cells. Alternatively, the enzyme can be obtained by a chemical synthesis based on the amino acid sequence in SEQ ID NO:2. The enzyme may be obtained by the method according to the present invention to be described hereinafter.

### Polynucleotide Sequence

The polynucleotide of the present invention is as described in (1), (2), (3) or (4) below:
(1) a polynucleotide comprising the nucleotide sequence shown in SEQ ID NO:1;
(2) a polynucleotide that hybridizes with the complement of the polynucleotide having a nucleotide sequence shown by SEQ ID NO:1 under stringent conditions, the polynucleotide encoding a heat-resistant DNA ligase having activity being not substantially decreased by heat treatment at 100°C for 1 hour;
(3) a polynucleotide encoding a polypeptide having at least about 90% sequence homology with the polypeptide set forth in SEQ ID NO:2, wherein the homologous polypeptide has a DNA ligase activity that is not substantially decreased by a heat treatment at 100°C for 1 hour; or
(4) a polynucleotide encoding any of the polypeptides of the present invention described above.

The polynucleotide of the present invention includes the polynucleotide itself having a given base sequence as well as a polynucleotide having a polynucleotide sequence complementary to the given base sequence, unless otherwise stated. The polynucleotide of the present invention also includes both DNAs and RNAs. The polynucleotide of the present invention further includes modified DNAs (for example phosphorothioate DNA, H-phosphorothioate DNA) and modified RNAs in the range capable of attaining the object of the present invention. The polynucleotide of the present invention further includes both single-stranded polynucleotides and double-stranded polynucleotides, and the double-stranded polynucleotide includes a DNA·RNA hybrid.

Examples of the phrase "stringent conditions" as used in the present invention include a temperature of 68°C in a standard hybridization solution, and a temperature of 42°C in a hybridization solution containing 50% formamide. These conditions are described in detail in the second edition, volume 2 of Molecular Cloning: A Laboratory Manual (Sambrook et al., 1990). Further details may be found in Jeon and Ishikawa, *FEBS Letters* 550:69-73 (2003), incorporated herein in its entirety.

The polynucleotide described in (2) is preferably a polynucleotide encoding a polypeptide comprising an amino acid sequence having 1 to about 200 deleted, substituted or added amino acids, particularly 1 to about 100 amino acids, in the amino acid sequence represented by SEQ ID NO:2. However, the DNA may be a polynucleotide encoding a polypeptide having an amino acid sequence modified in the range of 30% or less of the total amino acids in SEQ ID NO:2, so long as the amino acids belong to a non-conservative region between the DNA ligase regions.

The polynucleotides of the present invention include polynucleotides encoding polypeptides having at least about 70%, 75%, 80%, 85%, 90%, 91%, 92%, 93%, 94%, 95%, 96%, 97%, 98%, or 99% sequence homology with the polypeptide of SEQ ID NO:2. Preferably, the polynucleotides of the present invention encode a homologous polypeptide wherein amino acid changes in the homologous polypeptides occur in regions of the polypeptide of SEQ ID NO:2 that are not conserved among known DNA ligase polypeptides. Also preferably, the homologous polypeptides have a DNA ligase activity that is not substantially decreased by a heat treatment at 100°C for 1 hour.

The polynucleotides of the present invention can be isolated by hybridization from chromosome DNA libraries of thermophiles such as *Aeropyrum, Pyrococcus, Sufolobus, Thermoplasma, Thermoproteus, Mastigocladus, Bacillus, Synechococcus* and *Thermus* species using a probe designed based on SEQ ID NO:1. Or, the polynucleotides may be amplified by a PCR method using the chromosome DNA libraries of the thermophiles described above designed based on the base sequence shown in SEQ ID NO:1. The polynucleotide may be also obtained by chemical synthesis.

The modified polynucleotides in (2) or (3) can be prepared by a method known in the art such as chemical synthesis, gene engineering and induced mutation. The gene engineering method includes nucleotide-deleted introduction using an exonuclease, linker introduction, site-specific mutation introduction, and modification of base sequences by a PCR method using mutated primers to DNA ligase genes in (1).

### II. Recombinant Vector

The vector of the present invention is a recombinant vector comprising any of the polynucleotides (DNA) of the present invention as described above. A wide range of vectors known in the art may be used as the vector in which DNA of the present invention is integrated, and the vectors available include those for bacteria, yeast vectors and animals. A vector for bacteria is usually used considering the productivity of the enzyme. Examples of the vectors known in the art include *E. coli* vectors pBR322, pUC19 and pKK233-2; *Bacillus* species vectors pUB110, pC194, pE104, pTH15 and pBD16; yeast vectors Yip5, Yrp17 and Yep24; and animal vectors pUC18, pUC19 and M13mp18.

### III. Transformant

The transformant of the present invention is a host cell retaining the recombinant vector of the present invention as described above. Bacteria, yeast and animal cells may be used as hosts depending on the vectors. *Bacillus subtilis, Bacillus brevis,* yeast and fungi are preferable as the hosts for producing a large quantity of desired proteins. The cells may be transformed by a method known in the art such as a calcium phosphate method, protoplast method, electroporation method, spheroplast method, lithium acetate method, lipofection method and micro-injection method. The method used may be selected from these methods known in the art depending on the kind of host.

### IV. Method for producing DNA ligase

The method for producing the DNA ligase of the present invention comprises the steps of cultivating the host cell transformants of the present invention, and retrieving the DNA ligase produced by the transformants.

The cultivation conditions are not particularly restricted, and the host cells may be cultivated on a cultivation medium capable of growing the host cell, and under an appropriate temperature. While the cultivation time is different depending on other cultivation conditions, it is usually 12 to 24 hours. When induction expression vectors such as temperature-shift vectors and IPTG (isopropyl-b-D-thiogalactopyranose) induction vectors are used, the induction time may be 2 to 8 hours.

The DNA ligase is retrieved by breaking the cells by a cell breaking method known in the art such as ultrasonic treatment and surfactant treatment. The DNA ligase secreted in the culture medium may be retrieved when the host secretes DNA ligase.

For further purifying the retrieved DNA ligase, if necessary, protein purification methods known in the art such as centrifugation, slating out, solvent precipitation, dialysis, ultrafiltration, gel filtration, ion-exchange chromatography, affinity chromatography and reversed-phase liquid high performance liquid chromatography may be used in combination.

The DNA ligase of the present invention is preferably purified by heat treatment as one of the purification steps. The heat treatment is effective by usually applying it at a temperature of 10°C, particularly 15°C higher than the growth limit temperature of the host, and at a temperature of 80 to 95°C, which is the temperature at which about 60 to about 80% of the activity of the DNA ligase of the present invention remains, for 10 to 60 minutes. This treatment permits protein impurities produced by the host to be deactivated by denaturation without inactivating most of the desired DNA ligase. The denatured protein impurities can be precipitated by, for example, centrifugation of the purified enzyme solution at about 15,000 rpm for about 20 minutes after the heat treatment, although the method is not particularly restricted. This heat treatment may be applied at any stages of purification. This treatment enables the purity of the heat-resistant DNA ligase to be remarkably improved.

### Examples

While the present invention is described in more detail with reference to examples, the present invention is by no means restricted to these examples.

### Example 1 - Cloning of the DNA ligase gene of Aeropyrum pernix K1 strain

### i) Preparation of chromosome DNA

After dissolving 37.4 g of Bacto-marine culture medium (manufactured by Difco Laboratories) and 1.0 g of Na₂S₂O₃·5H₂O in 1 L of water, a culture medium was prepared by adjusting the pH to 7.0 to 7.2, and the medium was sterilized by heating under pressure. Super thermophile archaebacteriun *Aeropyrum pernix* K1 strain (deposited in the Institute of Physical and Chemical Research Japan with an accession number JCM9820) was seeded on this culture medium, and the cells were cultivated with shaking at 90°C for 3 days. The cells were collected by centrifugation of the culture medium at 5,000 rpm for 10 minutes.

The cells were sealed in an Insert agarose block (manufactured by FMC Co.) after washing with a 10-mM tris (pH 7.5)-1mM EDTA solution twice. Chromosomal DNA was separately prepared in the agarose block by treating the block with 1% N-lauroylsarcosine-1 mg/ml protease K solution. The separation conditions of the chromosomal DNA using the insert agarose block were in accordance with the manufacturer's instruction.

### ii) Amplification of the DNA ligase gene

DNA containing the base sequence in SEQ ID NO:1 (APE1094) was amplified by the PCR method. The PCR conditions were in accordance with the manufacturer's instruction. An oligonucleotide primer starting from base No. 1 (or starting from an initiation codon) in the DNA sequence shown in SEQ ID NO:1 in the sequence table was synthesized (5'-GGCTGTCTGGTTTTGGCTTCT-3'; SEQ ID NO:3) as a primer corresponding to the 5'-terminal. A primer corresponding to a primer from the 3'-terminal of base sequence to the downstream side of the base SEQ ID NO:1 in chromosomal DNA of *Aeropyrum pernix* K1 strain, or a primer that generates a BamHI site of a restriction enzyme in the amplified DNA, was also synthesized (5'-GTGAAGGGATCCTTACACCTGCTCCGC-3'; SEQ ID NO:4) as the primer corresponding to the 3'-terminal. After the PCR reaction, DNA was completely decomposed by treating it with the restriction enzyme BamHI at 37°C for 3 hours. Then, the DNA ligase gene was purified using a purification column kit.

### iii) Construction of a vector containing DNA ligase gene

After cutting and purification of vector pET-3d (manufactured by Novagen Co.) with the restriction enzyme Ncol, the terminal thereof was blunted using T4 DNA polymerase. The purified plasmid was cut with the restriction enzyme BamHI. Subsequently, plasmid pET-8c cut with BamHI was spliced with a DNA ligase gene cut with BamHI using T4 ligase by a reaction at 16°C for 16 hours. Competent cells of *E. coli* JM109 strain (manufactured by Takara Bio Co.) were transformed using spliced DNA. The transformant was selected using colonies formed on an LB agar plate containing 0.05 mg/ml of ampicillin as the selection marker. The DNA ligase gene-containing plasmid was extracted from the transformant by an alkaline method.

### Example 2 - Expression of the recombinant DNA ligase

### i) Preparation of transformant containing the DNA ligase gene

Competent cells (0.1 ml, 2,000,000 cfu) of E. *coli* Rosetta (DE) strain (prepared by Novagen Co.) and a solution of DNA ligase gene-containing plasmid DNA (0.002 ml, plasmid DNA 10 ng) were added to a 1.5-ml volume tube. After allowing the tube to stand on ice for 30 minutes, heat shock was applied to the cells at 42°C for 30 seconds. Then, the cells were cultivated with shaking at 37°C for 1 hour after adding 1 ml of SOC medium. Subsequently, the culture medium was applied on an LB agar plate containing ampicillin to obtain a transformant by cultivating at 37°C overnight.

### ii) Purification of DNA ligase

The transformant obtained was seeded on NZCYM medium containing ampicillin. After cultivation at 37°C until the absorbance at 600 nm reached 0.5, IPTG was added to enhance the amount of the plasmid followed by additional cultivation for 4 hours. The cells were collected by centrifugation of the culture medium at 8,000 rpm for 10 minutes.

A 50-mM potassium phosphate buffer solution (50 ml, pH 7.5) containing 1 mM DTT, 1 mM EDTA and 10 mM MgCl₂ was added to 2.8 g of the collected cells, and the cells were pulverized with ultrasonic waves for 3 minutes at an output of 90 W. The pulverized cell solution was centrifuged at 15,000 rpm for 30 minutes to obtain the supernatant.

After heating the supernatant at 85°C for 30 minutes to precipitate protein impurities, the supernatant was centrifuged at 15,000 rpm for 20 minutes to collect the supernatant. The collected supernatant was dialyzed against a 50 mM Tris-HCl buffer solution containing 15-mM MgCl₂ and 0.1 mM EDTA, and applied on a column of an anion-exchange chromatography resin, HitrapQ (manufactured by Pharmacia Co.), equilibrated with the same buffer solution for purification by ion-exchange column chromatography.

Active fractions were further dialyzed against 50 mM Tris-HCl buffer solution (pH 8.2) containing 150 mM NaCl and 15 mM MgCl₂, and subjected to gel filtration chromatography using a column packed with a gel filtration material, Sephacryl S-100 HR26/60 (manufactured by Pharmacia Co.), equilibrated with the same buffer solution. The active fractions contained a uniform standard sample that gave a single band by SDS-polyacrylamide gel electrophoresis.

The molecular weight of the enzyme of the present invention was found to be about 69 kDa from the results of gel filtration chromatography.

### Example 3 - Identification of DNA ligase of super thermophile archaebacteriun Aeropyrum pernix K1 strain

The DNA sequence of the DNA ligase gene of the super thermophile archaebacteriun *Aeropyrum pernix* K1 strain obtained in Example 1 is shown in SEQ ID NO:1 (APE1094). The amino acid sequence of the *Aeropyrum pernix* K1 strain obtained in Example 2 is shown in SEQ ID NO:2.

### Example 4 - Properties of the heat-resistant DNA ligase

Properties of the DNA ligase derived from the *Aeropyrum pernix* K1 strain obtained by the method above were evaluated. The activity of the DNA ligase was measured by a nick-closing assay or cohesive end ligation assay.

### Nick-closing assay

The DNA ligase activity was measured by the nick-closing assay (J. Bacteriology, vol. 182, p6424-6433, 2000). In more detail, 0.001 mg of each of the following three DNA primers AL1: 5'-phosphorylated 5'-TAAGCTCCGGATTGTCCGGGAGGTAAAGCCCTGAT-3' (SEQ ID NO:5), AL2: 5'-CACAGGAAGCTCTACAGGTACTCCG-3' (SEQ ID NO:6), and AL3: 5'-TGGTCATCAGGGCTTTACCTCCCGGACATTCCGGACCTTACGGAGTACCTGTAGAGCTTC CTGTGCAAGC-3' (SEQ ID NO:7) were mixed with the DNA ligase to a final concentration of 20 nM of, and 20 µL of the reaction solution (50 mM Tris-HCl (pH 7.5), 50 mM KCl, 10 mM MgCl₂, 5 mM DTT, 0.1 mM ATP). After reaction at 70°C for 10 minutes, the reaction solution was subjected to SDS-polyacrylamide gel electrophoresis, and the gel was stained with an ethidium bromide solution (1 mg/ml). Subsequently, the quantities of reaction products were relatively evaluated by measuring the concentrations of bands corresponding to oligonucleotides formed by splicing of the three nucleotides using a densitometer. The relative quantity of the reaction product showed the relative activity of the DNA ligase.

### Cohesive end ligation assay

The DNA ligase activity was measured by a cohesive end ligation assay (Extremophiles, vol. 5, p161-168, 2001). In more detail, λ-DNA cut with 0.5 µg of Hind-III, the DNA ligase with a final concentration of 100 nM and 20 µL of the reaction solution (50 mM Tris-HCl (pH 7.5), 50 mM KCl, 10 mM MgCl₂, 10 mM DTT, 1 mM ATP) were mixed, and the mixture was allowed to react at 37°C for 2 hours. Then, the reaction solution was subjected to agarose gel electrophoresis, and the gel was stained with an ethidium bromide solution (1 mg/ml). The relative quantity of the reaction product was evaluated by measuring the optical density of the bands corresponding to λ-DNA formed by ligation. The relative quantity of the reaction product showed the relative value of the DNA ligase activity.

### i) Optimum temperature

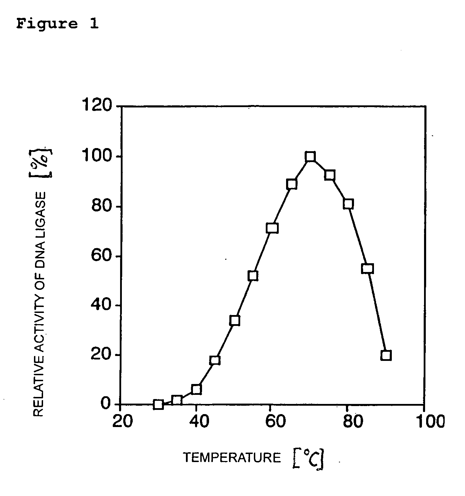

The DNA ligase activity was measured by a nick-closing assay and cohesive end ligation assay at 30 to 90°C. The relative activity of the enzyme is shown in Fig. 1. Fig. 1 shows that the optimum temperature of the enzyme of the present invention in the Tris-HCl buffer at pH 7.5 is 70°C.

### ii) Heat resistance

Three enzyme sample solutions were prepared by adding the DNA ligase to the reaction solution (50 mM Tris-HCl (pH 7.5), 10 mM MgCl₂, 5 mM DTT) so that the concentrations were 0.1 mg/ml, 1 mg/ml and 10 mg/ml, respectively (9 samples in total). Three samples with the respective concentrations were incubated at 100°C, 105°C and 110°C, respectively, for 1 hour to determine time-dependent residual activity ratios. The temperatures were adjusted to the desired temperatures of 100°C and 110°C by pressurizing.

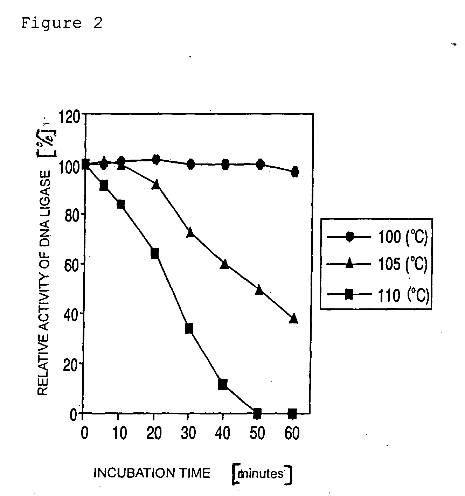

The residual activity ratios at each incubation temperature were approximately the same irrespective of the DNA ligase concentrations. The residual activity ratios of three samples having different DNA ligase concentrations were averaged for each incubation temperature. The results are shown in Fig. 2.

The enzyme of the present invention showed about 97% of the residual activity ratio after 1 hour by incubating at 100°C. The residual activity ratio was about 90% after incubating at 100°C for 12 hours, although the data are not shown in the specification. The residual activity ratio was about 100% after incubating for 10 minutes, while the residual activity ratio was about 85% after incubating for 20 minutes at 105°C. The residual activity ratio was about 80% after incubating for 10 minutes, while the residual activity ratio was about 65% after incubating for 20 minutes at 110°C.

### iii) Cofactor

### ATP/ATP

The DNA ligase activity was also measured using ADP in place of ATP in the cohesive end ligation assay and nick-closing assay described above. The results showed that approximately the same level of DNA ligase activity was obtained as when using ATP. It was shown that enzyme reactions are possible by using ADP in place of ATP in the enzyme of the present invention.

### Cations

The DNA ligase activities were measured using 10 mM MnCl₂, 10 mM CaCl₂ and 10 mM CoCl₂ in place of 10 mM MgCl₂. The DNA ligase activity was also measured without adding these cations. The results showed that, although the activity of the enzyme of the present invention was low when no divalent cations were added, the same levels of DNA activity as those obtained by adding MgCl₂ were obtained by adding any of MnCl₂, CaCl₂ and CoCl₂.

These results show that the enzyme of the present invention requires a divalent cation as a cofactor, and any of MgCl₂, MnCl₂, CaCl₂ and CoCl₂ are available as the divalent cations.

### Advantages

The present invention provides a heat-resistant DNA ligase capable of retaining high activity even after heat treatment at a high temperature.

The activity of the DNA ligase isolated from the super thermophile archaebacteriun *Aeropyrum pernix* K1 strain is not substantially decreased even after heat treatment at 110°C for 10 minutes, and the residual activity ratio of the enzyme is 80% or more by heat treatment at 110°C for 10 minutes. These results show that the enzyme of the present invention has quite a high heat stability. Since heat cycles of heating at 90 to 100°C and cooling to 50 to 65°C are usually repeated in LCR, a DNA ligase capable of maintaining high activity even after such heat treatment is required. Accordingly, the enzyme of the present invention can be favorably used as the DNA ligase used for LCR since the DNA ligase activity is maintained even after LCR heat treatment at 100°C for 1 hour.

A rapid treatment while chilling a reaction solution with ice has been required to avoid the enzyme from being deactivated in conventional DNA ligation methods. It is an advantage of the enzyme of the present invention that both strict chilling and rapid handling of the reaction solution are not required since the enzyme of the present invention has excellent stability at room temperature. In addition, since DNA ligation is carried out at a relatively high temperature, the effect of protein impurities may be reduced with high enzyme reaction efficiency.

Long-term storage is possible due to the stability of the enzyme of the present invention at room temperature.

The enzyme of the present invention is resistant to organic solvents due to its stable molecular structure, and the DNA ligase reaction is possible in organic solvents as well as in aqueous organic solutions in which enzymes are usually liable to be inactivated. Novel reactions related to DNAs may be expected given the facts described above.

The enzyme of the present invention is able to use ATP as a cofactor, and ADP can be utilized in place of ATP. ADP is more stable and less expensive than ATP. While the enzyme also requires divalent cations, any of Mg₂₊, Mn₂₊, Ca₂₊ and CO₂₊ are available as the divalent cations.

While the invention has been described in detail and with reference to specific embodiments thereof, it will be apparent to one of skill in the art that various changes and modifications can be made therein without departing from the spirit and scope thereof.

Sequence Listing

SEQ ID NO:1

Sequence (accession NO: APE1094 of Aeropyrum pernix K1)

gtggggtgtc tggttttggc ttctagctct gggggtgttg gaggcggtga catgcctttc 60
aagcccgtgg ctgaggcctt cgcctccatg gagaggataa cctctaggac gcagctcacc 120
ctcctcctca caaggctctt caagtccacg cccccggggg cgatcggtat tgtggtgtac 180
ctgatccagg ggaagctggg gcccgactgg aaggggctgc cggagctggg tgtcggggag 240
aagctgcttg taaaggccat agccctggct tacaaggcca ctgaggagag ggttgagagg 300
ctctacaagt ctgtaggcga cctggggagt gtggccgaga ggctgtcgcg ggagtaccgc 360
tcccgggctg ccagggccgt caccctggag gcgttcatgg cgggaggggg ggaggcgctg 420
actgtgagga gggtttacaa cacgctgtac aggatagcca tggcgcaggg tgaggggagc 480
agggacatca agcttaggct gctggccggc ctcctggcgg acgccgagcc cgtggaggcg 540
aagtatattg tgaggtttgt ggaggggagg ctgagggtgg gtgttgggga cgcgaccgtc 600
ctcgacgccc tcgccatggc cttcggcggc ggggcccacg cgaggcccgt tatagagagg 660
gcctacaacc tcagggccga cctaggctac atagcggagg tcgtggccag ggagggtgtt 720
gatgcgctga ggggtgtgaa gccccaggtc ggcgttccta taaggccgat gctggccgag 780
agggggaggg acccggctga gatactcagg aaggtggggg gcagggctgt cgtcgagtat 840
aagtacgatg gggagagggc gcagatacac aagaaggacg ggaggtcta catctactcg 900
aggaggcttg agaacataac caggatgttc cccgacgtgg ttgagatggc gaggaagggc 960
ctcaaagccg gggaggctat agtcgagggg gagatagtgg ccgtagaccc agacaactat 1020
gagatacagc ccttccaggt cctcatgcag aggaagagga agcacgacat acacagggtc 1080
atgagggagg tgcccgtcgc cgtcttcctc ttcgacgccc tctacgtgga cggcgaggac 1140
ctcacaagca aacccctccc cgagaggcgc aggaggctca aggagatagt tgtggagacg 1200

cccctctgga ggctggcgga gtccatcgag accagcgacc ccgaggagct gtggaccttc 1260

ttcctgaagg ccatagagga gggggccgag ggcgtcatgg tcaaggccgt ccacagggac 1320

tcagtctaca ccgcgggcgt aagggggtgg ctgtgggtca agctgaagag ggattacaag 1380

agcgagatga tggacacggt ggacctcgta gtggtgggcg ccttctacgg cagggggaag 1440

aggggcggga agctcagcag cctgctcatg gccgcctacg acccagacag ggacgtgttc 1500

cccaccgtct gcaaggtggc cacagggttc acggacgagg agctggacag gatgaacgag 1560

atgctgaaga agcacatcat acccaggaag cacccgaggg tagagtcgag gatagagcct 1620

gacgtgtggg tggagcccgc cctcgtggcg gagatactgg gcgccgagct caccctctca 1680

ccaatgcaca cctgctgcct caacactgtg aggccggggg tggggataag cataaggttc 1740

cccaggttca taaggtggag ggacgacaag agtccggagg acgcgacaac aacccacgag 1800

ctgctcgaga tgtacaagag gcagttgagg agggttgaag agccggcgga gcaggtgtag 1860

SEQ ID NO:2

Amino acid sequence (accession NO: APE1094 of Aeropyrum pernix K1)

Val Gly Cys Leu Val Leu Ala Ser Ser Ser Gly Gly Val Gly Gly Gly

1               5                   10                  15

Asp Met Pro Phe Lys Pro Val Ala Glu Ala Phe Ala Ser Met Glu Arg

            20                  25                  30

Ile Thr Ser Arg Thr Gln Leu Thr Leu Leu Leu Thr Arg Leu Phe Lys

        35                  40                  45

Ser Thr Pro Pro Gly Ala Ile Gly Ile Val Val Tyr Leu Ile Gln Gly

    50                  55                  60

Lys Leu Gly Pro Asp Trp Lys Gly Leu Pro Glu Leu Gly Val Gly Glu

65                  70                  75                  80

Lys Leu Leu Val Lys Ala Ile Ala Leu Ala Tyr Lys Ala Thr Glu Glu

                85                  90                  95

Arg Val Glu Arg Leu Tyr Lys Ser Val Gly Asp Leu Gly Ser Val Ala

            100                 105                 110

Glu Arg Leu Ser Arg Glu Tyr Arg Ser Arg Ala Ala Arg Ala Val Thr

        115                 120                 125

Leu Glu Ala Phe Met Ala Gly Gly Gly Glu Ala Leu Thr Val Arg Arg

    130                 135                 140

Val Tyr Asn Thr Leu Tyr Arg Ile Ala Met Ala Gln Gly Glu Gly Ser

145                 150                 155                 160

Arg Asp Ile Lys Leu Arg Leu Leu Ala Gly Leu Leu Ala Asp Ala Glu

                165                 170                 175

Pro Val Glu Ala Lys Tyr Ile Val Arg Phe Val Glu Gly Arg Leu Arg

            180                 185                 190

Val Gly Val Gly Asp Ala Thr Val Leu Asp Ala Leu Ala Met Ala Phe

        195                 200                 205

Gly Gly Gly Ala His Ala Arg Pro Val Ile Glu Arg Ala Tyr Asn Leu

    210                 215                 220

Arg Ala Asp Leu Gly Tyr Ile Ala Glu Val Val Ala Arg Glu Gly Val

225                 230                 235                 240

Asp Ala Leu Arg Gly Val Lys Pro Gln Val Gly Val Pro Ile Arg Pro

                245                 250                 255

Met Leu Ala Glu Arg Gly Arg Asp Pro Ala Glu Ile Leu Arg Lys Val

            260                 265                 270

Gly Gly Arg Ala Val Val Glu Tyr Lys Tyr Asp Gly Glu Arg Ala Gln
        275                 280                 285
Ile His Lys Lys Asp Gly Glu Val Tyr Ile Tyr Ser Arg Arg Leu Glu
    290                 295                 300
Asn Ile Thr Arg Met Phe Pro Asp Val Val Glu Met Ala Arg Lys Gly
305                 310                 315                 320
Leu Lys Ala Gly Glu Ala Ile Val Glu Gly Glu Ile Val Ala Val Asp
                325                 330                 335
Pro Asp Asn Tyr Glu Ile Gln Pro Phe Gln Val Leu Met Gln Arg Lys
            340                 345                 350
Arg Lys His Asp Ile His Arg Val Met Arg Glu Val Pro Val Ala Val
        355                 360                 365
Phe Leu Phe Asp Ala Leu Tyr Val Asp Gly Glu Asp Leu Thr Ser Lys
    370                 375                 380
Pro Leu Pro Glu Arg Arg Arg Arg Leu Lys Glu Ile Val Val Glu Thr
385                 390                 395                 400
Pro Leu Trp Arg Leu Ala Glu Ser Ile Glu Thr Ser Asp Pro Glu Glu
                405                 410                 415
Leu Trp Thr Phe Phe Leu Lys Ala Ile Glu Glu Gly Ala Glu Gly Val
            420                 425                 430
Met Val Lys Ala Val His Arg Asp Ser Val Tyr Thr Ala Gly Val Arg
        435                 440                 445
Gly Trp Leu Trp Val Lys Leu Lys Arg Asp Tyr Lys Ser Glu Met Met
    450                 455                 460
Asp Thr Val Asp Leu Val Val Val Gly Ala Phe Tyr Gly Arg Gly Lys

465                 470                 475                 480

Arg Gly Gly Lys Leu Ser Ser Leu Leu Met Ala Ala Tyr Asp Pro Asp

                485                 490                 495

Arg Asp Val Phe Pro Thr Val Cys Lys Val Ala Thr Gly Phe Thr Asp

            500                 505                 510

Glu Glu Leu Asp Arg Met Asn Glu Met Leu Lys Lys His Ile Ile Pro

        515                 520                 525

Arg Lys His Pro Arg Val Glu Ser Arg Ile Glu Pro Asp Val Trp Val

    530                 535                 540

Glu Pro Ala Leu Val Ala Glu Ile Leu Gly Ala Glu Leu Thr Leu Ser

545                 550                 555                 560

Pro Met His Thr Cys Cys Leu Asn Thr Val Arg Pro Gly Val Gly Ile

                565                 570                 575

Ser Ile Arg Phe Pro Arg Phe Ile Arg Trp Arg Asp Asp Lys Ser Pro

            580                 585                 590

Glu Asp Ala Thr Thr Thr His Glu Leu Leu Glu Met Tyr Lys Arg Gln

        595                 600                 605

Leu Arg Arg Val Glu Glu Pro Ala Glu Gln Val

    610                 615

SEQ ID NO:3

GGCTGTCTGGTTTTGGCTTCT

SEQ ID NO:4

GTGAAGGGATCCTTACACCTGCTCCGC

SEQ ID NO:5

TAAGCTCCGGATTGTCCGGGAGGTAAAGCCCTGAT

SEQ ID NO:6

CACAGGAAGCTCTACAGGTACTCCG

SEQ ID NO:7

TGGTCATCAGGGCTTTACCTCCCGGACATTCCGGACCTTACGGAGTACCTGTAGAGCTTCCTGTGCAAGC

## Claims

1. A heat-resistant DNA ligase, wherein said ligase has an activity that is not substantially decreased by heat treatment at 100°C for 1 hour.

2. The heat-resistant DNA ligase according to Claim 1, where said ligase further has properties comprising:
(a) an optimum activity at a temperature of 70°C or more;
(b) utilization of ATP or ADP as a cofactor; and
(c) utilization of Mg²⁺, Mn²⁺, Ca²⁺ or Co²⁺ as a cofactor.

3. The heat-resistant DNA ligase according to Claim 1 or 2, wherein said ligase is derived from *Aeropyrum pernix.*

4. A substantial pure polypeptide molecule comprising:
(a) a first polypeptide having the amino acid sequence set forth in SEQ ID NO:2; or
(b) a second polypeptide having the amino acid sequence set forth in SEQ ID NO:2, wherein said second polypeptide has one or more amino acid deletions, substitutions or additions, and further wherein said second polypeptide has DNA ligase activity that is not substantially decreased by heat treatment at 100°C for 1 hour.

5. An isolated polynucleotide molecule comprising;
(a) a first polynucleotide having the nucleotide sequence set forth in SEQ ID NO:1;
(b) a second polynucleotide that hybrids with a complement of the nucleotide sequence set forth in SEQ ID NO:1 under stringent conditions, wherein said second polynucleotide encodes a heat-resistant DNA ligase having activity that is not substantially decreased by heat treatment at 100°C for 1 hour;
(c) a third polynucleotide that encodes a polypeptide having at least about 90% sequence homology with the polypeptide set forth in SEQ ID NO:2, wherein the polypeptide encoded by said third polynucleotide is a heat-resistant DNA ligase having activity that is not substantially decreased by heat treatment at 100°C for 1 hour; or
(d) a third polynucleotide encoding a polypeptide according to Claim 4.

6. A recombinant vector comprising a polynucleotide molecule according to Claim 5.

7. A host cell transformed with a recombinant vector according to Claim 6.

8. A method for producing a heat-resistant DNA ligase, comprising (a) culturing a host cell according to Claim 7 under conditions such that said host cell expresses said heat-resistant DNA ligase, and (b) collecting the heat-resistant DNA ligase so expressed.

9. The substantial pure polypeptide molecule according to claim 4, wherein said second polypeptide has at least about 90% sequence homology with the amino acid sequence set forth in SEQ ID NO:2

10. An isolated polynucleotide molecule comprising the nucleotide sequence set forth in SEQ ID NO:1.

11. An isolated polynucleotide molecule that encodes the amino acid sequence set forth in SEQ ID NO:2.

12. A substantial pure polypeptide molecule comprising the amino acid sequence set forth in SEQ ID NO:2.
